(19) Europäisches Patentamt
European Patent Office
Office européen des brevets

(11) EP 4 344 620 A1

(12) EUROPEAN PATENT APPLICATION

(43) Date of publication:
03.04.2024 Bulletin 2024/14

(21) Application number: 22198917.1

(22) Date of filing: 30.09.2022

(51) International Patent Classification (IPC):
A61B 5/00 $^{(2006.01)}$    A61B 18/00 $^{(2006.01)}$

(52) Cooperative Patent Classification (CPC):
A61B 5/6853; A61B 18/1492; A61B 2018/0022;
A61B 2018/00285

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• KOLEN, Alexander
  Eindhoven (NL)
• SHULEPOV, Sergei Y.
  Eindhoven (NL)
• GLASSNER, Lior Shlomo
  5656AG Eindhoven (NL)

(74) Representative: Philips Intellectual Property &
Standards
High Tech Campus 52
5656 AG Eindhoven (NL)

(54) **DETECTING OCCLUSION OF ANATOMICAL CAVITY**

(57)    A mechanism for detecting the presence/absence of an occlusion of an anatomical cavity by an interventional device, and therefore the presence/absence of a leak around the interventional device within the anatomical cavity. A plurality of electrical measurements between different electrode pairs are obtained, each electrode pair containing a device electrode carried by the interventional device and an external electrode positioned on the subject. The electrical measurements are processed to derive an occlusion indicator.

FIG. 2

Processed by Luminess, 75001 PARIS (FR)

**Description**

FIELD OF THE INVENTION

The present invention relates to techniques for detecting occlusion, by an interventional device, of an anatomical cavity.

BACKGROUND OF THE INVENTION

Ablation systems are commonly used to ablate the bounds of an anatomical cavity. An ablation system typically comprises a catheter assembly that carries an ablation balloon. The ablation balloon is moved to a desired occlusion location to occlude the anatomical cavity. The ablation balloon is then controlled or configured to perform ablation against the bounds of the occluded portion of the anatomical cavity.

To achieve appropriate ablation for meeting a clinical need, it is necessary for the ablation balloon to completely occlude the anatomical cavity at the desired occlusion location before and during the ablation procedure. Failure to completely occlude the anatomical cavity would result in incomplete ablation at the relevant location, which would not achieve a desired medical effect.

There is therefore a desire to identify successful occlusion of the anatomical cavity and to improve the monitoring and identification of continued occlusion at the anatomical cavity.

Of course, there are other reasons for occluding an anatomical cavity. For instance, some occlusion devices may be used to close or block anatomical cavities such as blood vessels to an appendage, which can be used to perform treatment on the appendage without blood loss.

SUMMARY OF THE INVENTION

The invention is defined by the claims.

According to examples in accordance with an aspect of the invention, there is provided an occlusion detection system for detecting occlusion of an anatomical cavity of a subject by an interventional device, which carries one or more device electrodes, using two or more external electrodes positioned on the surface of the subject.

The occlusion detection system comprises: an input interface configured to receive at least two electrical measurements, each electrical measurement being responsive to a change in an electrical property between a different electrode pair compared to any other electrical measurement, where each electrode pair comprises a device electrode and an external electrode; and a data processor configured to process the at least two electrical measurements to generate an occlusion indicator that indicates the presence or absence of occlusion of the anatomical cavity by the interventional device.

The present disclosure provides a mechanism for generating an occlusion indicator for an interventional device (e.g., a catheter or the like). The occlusion indicator is generated using two or more electrical measurements. Each electrical measurement is a measure of an electrical property between a device electrode (e.g., catheter electrode) and an external electrode (e.g., a body or patch electrode). Different measurements represent different pairings of such electrodes.

This approach significantly increases the number of electrical measurements that are made for determining occlusion. This allows more mathematical signal enhancements methods to be used, which can improve the accuracy and quality of the occlusion indicator and in case of a leak a more accurate estimation of the orientation or position of the leak.

It will be appreciated that the occlusion indicator similarly indicates the absence or presence of any leaks in the anatomical cavity (i.e., any failures to occlude). Put another way, detecting the absence of occlusion is identical to detecting the presence of a leak.

In some examples, the electrical property is an impedance between the electrode pair. In other examples, the electrical property is a voltage difference between the electrode pair. In yet other examples, the electrical property is an impedance difference, being a difference between: an impedance between the device electrode of the electrode pair and a reference electrode; and an impedance between the external electrode of the electrode pair and the reference electrode.

The at least two electrical measurements may comprise one or more sets of electrical measurements, wherein the device electrode in the electrode pair for each electrical measurement in the same set of electrical measurements is the same and the external electrode in the electrode pair for each electrical measurement in the same set of electrical measurements is different.

This approach makes use of multiple external electrodes in the production of the electrical measurements. This improves the occlusion detection at any single device electrode, which can provide more accurate and/or specific identification of any occlusions and/or leaks.

The one or more sets of electrical measurements may comprises two or more sets of electrical measurements, wherein the device electrode in the electrode pairs of different sets of electrical measurements is different.

The occlusion indicator may indicate, if occlusion is absent (and thereby resulting in a leakage), the location of the leakage in the anatomical cavity.

In some examples, the location of each external electrode on the surface of the subject relative to one another is predetermined; and the data processor is configured to determine, if occlusion is absent resulting in a leakage, a location of the leakage in the anatomical cavity, with respect to the two or more external electrodes, using a trilateration technique.

The at least two electrical measurements may comprise at least four electrical measurements.

In some examples, the occlusion indicator indicates the amplitude of any leakage past the interventional device in the anatomical cavity.

The data processor may be configured to generate the occlusion indicator by performing a process that includes: monitoring the electrical measurements over a period of time; and generating the occlusion indicator responsive to changes in the electrical measurements over the period of time.

In some examples, the data processor is configured to generate the occlusion indicator by performing a process that includes summing or averaging the electrical measurements.

Optionally, the input interface is configured to receive, for each electrical measurement, an initial electrical measurement sampled at an earlier point in time; and the data processor is configured to normalize each electrical measurement using the corresponding initial electrical measurement.

There is also proposed a computer-implemented method for detecting occlusion of an anatomical cavity of a subject by an interventional device, which carries one or more device electrodes, using two or more external electrodes positioned on the surface of the subject.

The computer-implemented method comprises: receiving at least two electrical measurements, each electrical measurement being responsive to a change in an electrical property between a different electrode pair compared to any other electrical measurement, where each electrode pair comprises a device electrode and an external electrode; and processing the at least two electrical measurements to generate an occlusion indicator that indicates the presence or absence of occlusion of the anatomical cavity by the interventional device.

The skilled person would be readily capable of adapting any herein disclosed computer-implemented method to perform the steps carried out by any herein disclosed processing system, and vice versa.

It will be appreciated that any herein proposed method is entirely passive, and does not require any direct interaction with the subject or individual. Rather, historic data (e.g., previously acquired) can be processed. In other words, the method can be entirely performed by a processing system that does not directly monitor or interact with the subject or individual.

There is also proposed a computer program product comprising computer program code means which, when executed on a computing device having a processing system, cause the processing system to perform all of the steps of any herein proposed computer-implemented method.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates an interventional device in the form of a balloon therapy catheter;
Figure 2 illustrates a scenario having an interventional device and a set of external electrodes;
Figure 3 illustrates a system having an occlusion detection system 250 according to an embodiment; and
Figure 4 illustrates a computer-implemented method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for detecting the presence/absence of an occlusion of an anatomical cavity by an interventional device, and therefore the presence/absence of a leak around the interventional device within the anatomical cavity. A plurality of electrical measurements between different electrode pairs are obtained, each electrode pair containing a device electrode carried by the interventional device and an external electrode positioned on the subject. The electrical measurements are processed to derive an occlusion indicator.

The present invention relies upon the recognition that more accurate or more contextually relevant information can be determined using such a plurality of electrical measurements.

Particularly advantageous embodiments make use of a plurality of external electrodes, which provides the capacity to further improve the accuracy of the occlusion detection and/or to identify the location/orientation of any leaks with respect to the interventional device.

Approaches can be used in any scenario in which the occlusion of an anatomical cavity by an interventional device is desired, e.g., for performing ablation or blocking blood flow to a particular portion of the anatomy.

The present disclosure provides an approach for detecting occlusion of an anatomical cavity of a subject by an interventional device. The interventional device carries one or more device electrodes, as well as any components necessary for occluding the anatomical cavity.

One example of an interventional device that can be used to occlude an anatomical cavity is a balloon therapy catheter assembly, which occludes an anatomical cavity using a balloon before performing therapy (such as ablation) on the occluded anatomical cavity. Where the balloon is used to perform ablation, it is labelled an ablation balloon.

Other examples of interventional device used to occlude an anatomical cavity will be apparent to the skilled person. For instance, some interventional devices may be configured for occluding or restricting blood flow to an appendage of a subject, e.g., for performing therapy or surgery on the appendage.

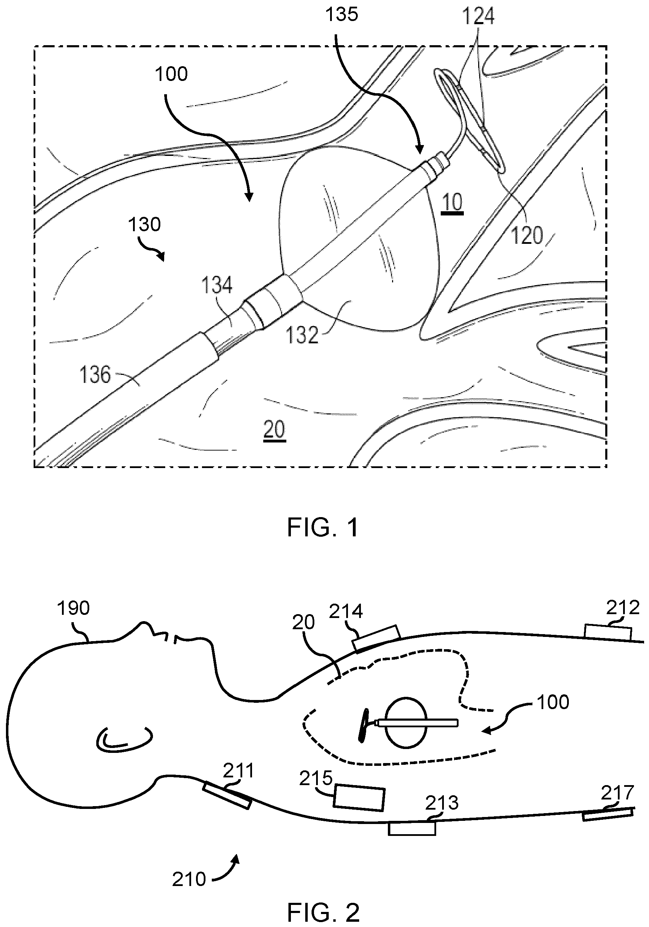

Figure 1 provides an exemplary embodiment of an interventional device 100, embodied as a balloon therapy catheter assembly, e.g., for use in ablation therapy. This is provided for the sake of contextual understanding only, and the skilled person would readily realize other forms of interventional device that could be used for the present invention.

The interventional device 100 is configured for performing occlusion within an anatomical cavity 20. As later explained, the interventional device 100 also carries one or more device electrodes 124.

To perform occlusion of or within an anatomical cavity, the interventional device here includes a balloon therapy catheter 130 having an inflatable balloon 132 (shown in inflated condition) that can be positioned at a desired occlusion location of an anatomical cavity. Typically, the inflatable balloon is maneuvered (in a deflated condition) to the desired occlusion location, where it can then be inflated to occlude the anatomical cavity at the desired occlusion location. The inflatable balloon 132, and therefore the interventional device, is therefore configured to controllably occlude a portion of the anatomical cavity.

Here, the desired occlusion location is exemplified as an ostium of a PV 10 within an overall anatomical cavity 20 representing the left atrium (LA) and PVs connected thereto of heart of a subject. Other suitable anatomical cavities and desired occlusion locations will be apparent to the skilled person.

The inflatable balloon 132 is attached to a distal portion of a flexible elongate member 134 of the balloon therapy catheter 130. The elongate member 134 has a member tip 135 at a distal position to the balloon 132.

Although not essential for use with the proposed inventive concept, the inflatable balloon may be an ablation balloon configured to perform ablation at the bounds of the occluded portion of the anatomical cavity.

In the illustrated example, the inflatable balloon 132 is a cryoballoon configured to be inflated and then at least partially filled with a cooling fluid (refrigerant) to cool the cryoballoon to a temperature (e.g., below -65° C) that causes an electrically-isolating lesion or firewall in the tissue when in contact with the boundary or bounds of the occluded portion of the cavity. For example, the cryoballoon 132 may be in fluid communication with a source or reservoir of cooling fluid via one or more fluid lines positioned within the flexible elongate member 134. Further, the cryoballoon 132 may be in fluid communication with an air or gas source, e.g., for balloon inflation, via one or more fluid lines positioned within the flexible elongate member 134.

In an alternative example, rather than a cryoballoon, the inflatable balloon 132 could mount a set of one or more ablation electrodes. Such ablation electrodes can be controlled for providing ablation energy in the form of RF power. Further details regarding suitable catheters and assemblies can be found in, for example, U.S. Patent No. 6,002,955, titled "Stabilized Electrophysiology Catheter and Method for Use," the entirety of which is hereby incorporated by reference.

The present invention makes use of one or more device electrodes 124, particularly data sampled at the one or more device electrodes. Device electrodes are any electrodes carried by the interventional device.

In the illustrated approach, the device electrode(s) 124 is/are carried by an electrophysiology (EP) catheter 120 that is slidably coupled with the balloon therapy catheter 130 of the interventional device (i.e., the balloon therapy catheter assembly).

In the illustrated example, the flexible elongate member 134 comprises a lumen extending from its proximal end (not shown) to its distal tip 135 and is suitable for slidably receiving such an electrophysiology (EP) catheter 120. The EP catheter comprises one or more (e.g. a plurality of) separately addressable electrodes 124, which can be used as the device electrode(s), disposed along a distal tip thereof. The EP catheter is extendible from or retractable into the lumen via the (distal) tip 135 of the flexible member 134. The EP catheter may be removed from the member 134 entirely if needed, e.g., to disassemble the catheter assembly 100.

In this way, the EP catheter 120 comprises one or more (e.g., a plurality of) mutually electrically separated electrodes 124, or device electrodes, disposed along an elongate catheter tip member 126.

In the illustrated example, the EP catheter 120 carries three electrodes. However, in other examples, the EP catheter can carry a single device electrode. However, the catheter 120 can include other numbers of electrodes, including 1, 2, 4, 6, 8, 14, 15, 20, 30, 60, or any other suitable number of electrodes. More than 10 electrodes are preferred and more than 15 are even more preferred.

In an alternative approach, where the inflatable balloon mounds a set of one or more ablation electrodes, the ablation electrode(s) could be used as the device electrode(s). In such approaches the EP catheter 120 does not need to be used and could be omitted.

The elongate member 134 may be movably disposed within a flexible steering sheath 136. When the balloon is in a deflated state the whole of the member 134 including the deflated balloon may be retracted into and through the sheath 136. Of course, there may be no need to deflate a sufficiently flexible balloon. When protruding out a distal end of a sheath 136, the balloon may be inflated as shown.

The flexible steering sheath 136 may comprise pull wires to be operated by a user to steer the sheath and guide the balloon therapy catheter as well as the EP catheter if present.

The movement of different catheters and sheaths may be controlled by a user using a catheter control device or handle. The handle is not shown in Figure 1 and such control is known in the art.

For example, if present, the EP catheter 120 may be controllable manually or via an automated mechanism to be retractable within and extendable from the lumen of the member 134 of the balloon therapy catheter 130. It may even be entirely removed from the lumen and for example replaced with a guide wire or other wire or catheter. The EP catheter 120 and therewith its electrodes may thus be moved relative to the inflatable balloon or member tip 135 for example. Likewise, the member 134 may be controlled by the handle and moved with respect to the sheath 136.

If present, the EP catheter 120 is preferably positioned distally of the balloon 132 during a therapy procedure, and may be biased, shaped, or otherwise structurally configured to assume a shape, such as a circular one in which the electrodes are spaced within one plane or a spiral one in which the electrodes are spaced from one another about one or more planes. For example, the elongate tip member can be similar to a spiral mapping catheter (SMC) in which electrodes are distributed along an elongate tip member having assumed or configurable to adopt a spiral configuration.

For the sake of contextual understanding, an example procedure for performing ablation using the illustrated interventional device 100 is hereafter described.

Using the controls described herein above, in some embodiments, a following procedure can be used. First, the sheath 136 having retracted therein the balloon therapy catheter with balloon in deflated state and the EP catheter 120 is first introduced into the LA via known procedures to guide the balloon and EP catheter 120 to the ablation site. Alternatively, a guidewire taking the place of the EP catheter may first be introduced in the PV over which the balloon therapy catheter including the flexible member 134, deflated balloon and sheath 136 are guided towards the LA before the guide wire is replaced with the EP catheter. Once the balloon therapy catheter 130 is in the LA, the flexible member 134 may be extended from the sheath 136 to expose the inflatable balloon 132 in order to inflate it as shown in Figure 1 The EP catheter 120 may now be extended from the member 134 out of the tip 135 to be spiral or lasso shaped and positioned in the PV. Once positioned, the member 134 may be manipulated to bring the inflated balloon towards the PV ostium so that a full circumference of the balloon 132 is in contact with an entire circumference of the PV 10 near or at the ostium. In this way, a full occlusion of the PV at the ostium may be achieved and by cooling of the balloon (or appropriate activation/control of electrodes positioned on the balloon) the ablation can be delivered around the entire circumference with an increased likelihood that the ablation results in complete electrical isolation.

Examples of balloon therapy catheters manufactured by Medtronic™ are the Arctic Front™ Family of Cardiac Cryoablation Catheters and/or the FlexCath™ Advance Steerable Sheath. Examples of EP catheters that work with the aforementioned examples manufactured by the Medtronic™ are the **Achieve™ and Achieve Advance™ Mapping Catheters Families. However, other types of such catheters for example from other manufacturers may be used in conjunction with the current mechanism.**

From the foregoing, it will be apparent that there is a desire to identify when occlusion (and particularly complete occlusion) of the anatomical cavity has occurred. This can be used to ensure that ablation is correctly performed around the bounds of the anatomical cavity.

However, the benefits of identifying occlusion of an anatomical cavity are not exclusive to an ablation use-case scenario, and it may be desirable to detect occlusion by other forms of interventional device that carry

**one or more device electrodes.**

The present invention relates to an approach for detecting occlusion of an anatomical cavity of a subject by an interventional device. Proposed approaches make use of external electrodes positioned on the surface of the subject, e.g., on the skin of the subject.

Figure 2 conceptually illustrates a scenario in which external electrodes 210 are positioned on the surface of a subject 190. The external electrodes 210 are located at different positions on the surface of the subject 190.

The external electrodes 210 may, for instance, comprise an array of external electrodes 211, 212, 213, 214, 215, 217 that are positioned about the subject 190. In particular, the external electrodes may comprise body patches that are coupled to the subject 190.

These external electrodes 210 may, for instance, be electrodes used for performing an electrocardiogram (ECG), as is well known in the art. In another example, the external electrodes may be electrodes usable for tracking the location of the interventional device 100 within the anatomical cavity 20. Approaches for tracking the location of an interventional device 100 within an anatomical cavity using external electrodes 210 are known in the art, for instance, as described in the International Patent Application having publication no. WO 2022/144198 A1.

The proposed approach makes use of at least two electrical measurements. Each electrical measurement is responsive to a change in an electrical property between a different electrode pair compared to any other electrical measurement. Each electrode pair comprises a device electrode (carried by the interventional device 100) and an external electrode (mounted on a surface of the subject 190).

The at least two electrical measurements are then generated to produce an occlusion indicator that indicates the presence or absence of occlusion of the anatomical cavity by the interventional device. It will be appreciated that an occlusion indicator also provides information on the presence or absence of any leaks past the interventional device.

The present invention firstly recognizes that an electrical measurement between a device electrode and external electrode is responsive to an occlusion state in the vicinity of the device electrode. The present invention also recognizes that using a plurality of such electrical measurements provides greater flexibility and contextual information for determining the overall occlusion status of the anatomical cavity by the interventional device (e.g., to detect the presence, amplitude and/or location of any leaks). The use of multiple electrical measurements can also be used to improve a signal-to-noise ratio of the occlusion indicator.

The proposed approach also makes use of external electrodes that are already positioned on the surface of the subject for other clinically relevant assessment and/or analysis. This facilitate the integration of the proposed approach into existing systems without the need for substantive amounts of additional circuitry or equipment.

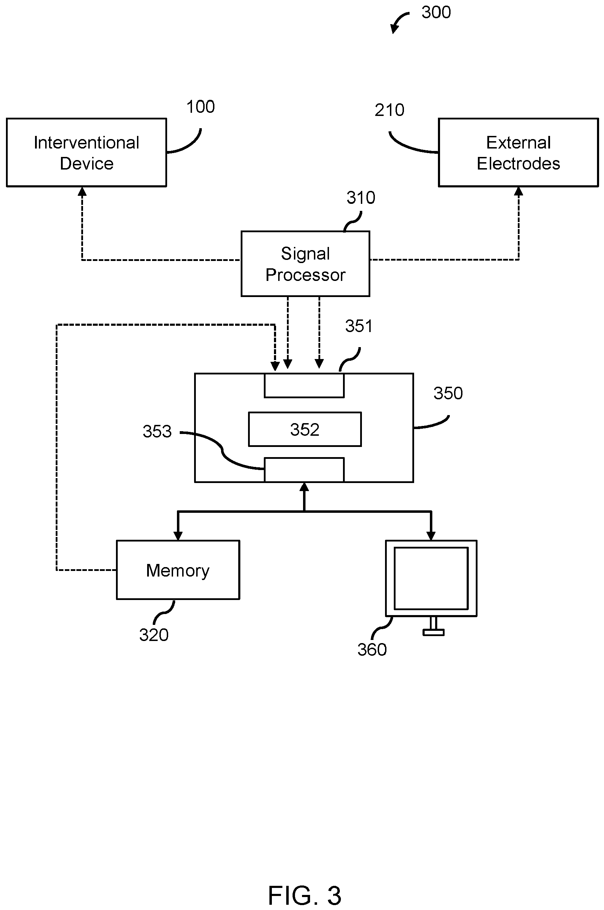

Figure 3 is a block diagram representation of a system 300 comprising an occlusion detection system 350 according to an embodiment.

The system 300 also comprises the interventional device 100 and the external electrodes 210. As previously explained, the interventional device is configured for occluding a portion of an anatomical cavity (e.g., using an inflatable balloon) and carries one or more device electrodes (e.g., carried by an EP catheter or mounted on the inflatable balloon).

The occlusion detection system 350 is configured for detecting occlusion of an anatomical cavity of a subject by an interventional device 100, which carries one or more device electrodes, using two or more external electrodes 210 positioned on the surface of the subject.

The occlusion detection system 350 comprises an input interface 351. The input interface is configured to receive at least two electrical measurements, each electrical measurement being responsive to a change in an electrical property between a different electrode pair compared to any other electrical measurement, where each electrode pair comprises a device electrode and an external electrode.

Thus, the input interface 351 receives, for each of a plurality of electrode pairs, an electrical measurement. The electrical measurement is responsive to a change in an electrical property. Each electrode pair comprises a device electrode and an external electrode, wherein each pair is different.

Examples of electrical properties include an impedance or a voltage difference. Other, and more detailed, examples are provided later in this disclosure.

The electrical measurements may, for instance, be obtained from a signal processor 310 that processes signals at any device electrode carried by the interventional device 100 and the external electrodes 210. The signal processor forms part of the system 300.

In particular, the signal processor may be configured to process any raw signals at such electrodes, e.g., to perform filtering, sampling and/or digitizing of such signals, using to produce the electrical measurements. The signal processor 310 may (electrically) connect to each electrode and process the raw signals at the electrodes to produce the electrical measurements for digital processing by a processing system or other similar device. The signal processor 310 may therefore comprise appropriate circuitry for performing such functions, e.g., filtering circuitry, sampling circuitry and/or digitizing circuitry (e.g., an analogue-to-digital converter).

The signal processor may also be configured to control electrical signals provided to the electrodes (i.e., the device electrode(s) and the external electrodes). For instance, electrical signals may be controlled to provide a current between different pairs of electrodes in order to monitor an impedance between electrodes in a same pair. Other, and more detailed, approaches for generating electrical measurements are provided later in this disclosure.

Alternatively, the electrical measurements may be obtained from a memory or storage unit 320, e.g., that stores pre-processed electrical measurements.

Thus, the occlusion detection system 350 does not need to directly interact with the subject.

The occlusion detection system 350 also comprises a data processor 352 configured to process the at least two electrical measurements to generate an occlusion indicator that indicates the presence or absence of occlusion of the anatomical cavity by the interventional device.

The occlusion detection system 350 may comprise an output interface 353. The output interface may be configured to provide output data responsive to the generated occlusion indicator.

In one example, the output data is used to control a visual representation provided by a display 360. In particular, the output data may be used to control the display to provide a visual representation of the occlusion indicator.

In another example, the output data is stored in a memory or storage unit 320. For instance the output data may carry or contain the occlusion indicator that is stored in the memory or storage unit, e.g., for later retrieval.

Various types of electrical measurements may be received by the input interface and processed by the data processor. As previously explained, each electrical measurement is a measure of an electrical property between a device electrode and an external electrode.

Each electrical measurement may be generated or produced by the signal processor 310. The signal processor may be configured to generate and/or monitor electrical signals in each pair of device and external electrodes.

In one example, each electrical measurement is an impedance between the electrode pair. An impedance Z can be calculated by sampling a voltage at each electrode in the electrode pair, and dividing by the current between the electrode pair. Thus, the following equation can be used:

$$Z = \frac{V_{dev} - V_{ex}}{I_{dev,ex}} \qquad (1)$$

where $V_{dev}$ is the voltage at the device electrode (e.g., with respect to an earth or reference voltage), $V_{ex}$ is the voltage at the external electrode and $I_{dev,ex}$ is the (electrical) current between the device and external electrode.

To distinguish between the current flow from a particular device electrode or external electrode, each electrode pair may be associated with a particular frequency or temporal (modulation) pattern. Thus, each pair of electrodes may be associated with a different frequency or temporal/modulation pattern. In particular, a current at a particular frequency or (modulation) pattern may be induced between the two electrodes in the pair. The magnitude of this current at the particular frequency/pattern and voltage(s) may be used to determine the impedance between the two electrodes.

In another example, each electrical measurement is a voltage difference between the electrode pair. Approaches for determining a voltage difference between a pair of electrodes (i.e., an electrode pair) are well known to the skilled person.

In yet another example, each electrical measurement is a difference of impedances, namely a difference between a first impedance (between the device electrode and a reference electrode) and a second impedance (between the external electrode and the same reference electrode).

A difference of impedances $Z_d$ can be calculated using the following equation.

$$Z_d = \frac{V_{dev} - V_{ref}}{I_{dev,ref}} - \frac{V_{ex} - V_{ref}}{I_{ex,ref}} \qquad (2)$$

where $V_{dev}$ is the voltage at the device electrode (e.g., with respect to an earth or reference voltage), $V_{ex}$ is the voltage at the external electrode, $V_{ref}$ is the voltage at a reference electrode, and $I_{dev,ref}$ is the current between the device and reference electrode and $I_{ex,ref}$ is the current between the external and reference electrode.

All of the above-described electrical measurements have been identified as being responsive to, i.e., changing in response to, a success/failure of occlusion and/or amount of leakage past the device electrode that is used to generate the electrical measurement. Thus, it is possible to generate an occlusion indicator using such electrical measurements.

The at least two electrical measurements may comprise one or more sets of electrical measurements, in which the device electrode in the electrode pair for each electrical measurement in the same set of electrical measurements is the same; and the external electrode in the electrode pair for each electrical measurement in the same set of electrical measurements is different.

Thus, each set of electrical measurements comprises a plurality of electrical measurements between a same device electrode and different external electrodes.

Thus, the electrical measurements preferably include at least a first electrical measurement (between a first external electrode and a first device electrode) and a second electrical measurement (between a second, different external electrode and the first device electrode). Of course, the electrical measurements may include additional electrical measurements.

The one or more sets of electrical measurements may comprises two or more sets of electrical measurements, in which the device electrode in the electrode pair for each electrical measurement of different sets of electrical measurements is different.

In this way, each set of electrical measurements may be associated with a particular device electrode. This allows, for instance, the determination of whether or not there is occlusion in the vicinity of each device electrode. As each device electrode will naturally be at a different position on the interventional device, this allows for detection of occlusion/leakage at different positions around the interventional device (e.g., the location and/or orientation of a leak, if present).

This can be exploited to perform localization or location identification of an occlusion/leak. In particular, the location of each device electrode may be determinable (e.g., using an electrode tracking technique such as those described by WO 2022/144198 A1). As both the location of the device electrode and whether or not there is an occlusion in the vicinity of the device electrode can be determined, it is possible to generate an indicator of the location of a leak and/or occlusion.

Preferably, each set of electrical measurements comprises more than two electrical measurements (i.e., there are more than two external electrodes). For instance, each set of electrical measurements may comprise 2, 4, 6, 8, 12 or more electrical measurements. This approach increases the effect and accuracy of any later processing steps that make use of the electrical measurements to generate the occlusion indicator.

The external electrodes used to generate the electrical measurements may, for instance, be electrodes used for performing an electrocardiogram (ECG) or electrodes usable for tracking the location of the interventional device within the anatomical cavity. The number of external electrodes, and therefore electrical measurements in a set of electrical measurements, may be defined by the (original) function or purpose of the external electrodes.

The number of sets of electrical measurements may be defined by the number of device electrodes carried by the interventional device (e.g., the number of individually addressable device electrodes). In particular, a set of electrical measurements may be defined or received for each individually addressable device electrode carried by the interventional device.

The number of sets may be 1, 2, 4, 6, 8, 14, 15, 20, 30, 60, or any other suitable number of sets. More than 10 sets are preferred and more than 15 are even more preferred.

Example approaches performed by the occlusion detection system to generate the occlusion indicator are hereafter described.

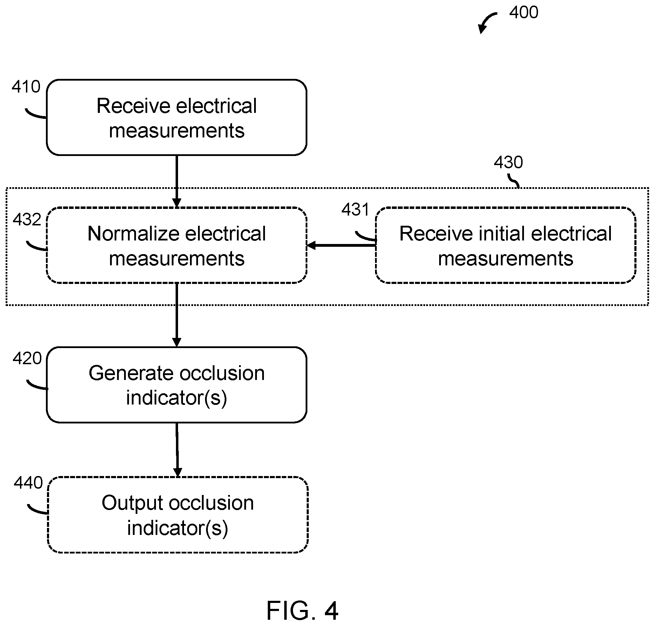

Figure 4 illustrates a computer-implemented method 400 that is performed by the occlusion detection system.

The method 400 comprises a step 410 of receiving at least two electrical measurements, each electrical measurement being responsive to a change in an electrical property between a different electrode pair compared to any other electrical measurement, where each electrode pair comprises a device electrode and an external electrode.

As previously explained, step 410 is performed by the input interface of the occlusion detection system.

The method 400 also comprises a step 420 of processing the at least two electrical measurements to generate an occlusion indicator that indicates the presence or absence of occlusion of the anatomical cavity by the interventional device. Step 420 is performed by the data processor of the occlusion detection system.

Step 420 may, for instance, comprise generating a plurality of occlusion indicators, e.g., an occlusion indicator for each device electrode that contributes to or is associated with the received electrical measurements. In particular, if the electrical measurements comprises a plurality of sets of electrical measurements, as previously described, then an occlusion indicator may be generated for each set.

Various types of data or information can be generated or determined by processing the at least two electrical measurements. A non-exhaustive number of example types of data or information are hereafter described, for the purposes of improved understanding.

In a first example, the electrical measurements comprise a first set of electrical measurements. The device electrode in the electrode pair for each electrical measurement in the first set of electrical measurements is the same. The external electrode in the electrode pair for each electrical measurement in the first set of electrical measurements is different.

In this first example, the occlusion indicator may be generated by averaging or summing the first set of electrical measurements to produce the occlusion indicator. It is recognized that an electrical measurement in itself will provide information about occlusion (as it changes responsive to occlusion of the anatomical cavity). Averaging or summing the electrical measurements provides an occlusion indicator with increased resilience and signal-to-noise ratio.

Thus, in this approach, a single occlusion indicator is produced having a value that changes responsive to a predicted occlusion or non-occlusion (i.e., leak) in the vicinity of the device electrode associated with the first set of electrical measurements.

In a variation of the first example, the electrical measurements comprise a plurality of sets of electrical measurements. The device electrode in the electrode pair for each electrical measurement in the first set of electrical measurements is the same. The external electrode in the electrode pair for each electrical measurement in the first set of electrical measurements is different. Thus, each of a plurality of device electrodes may be associated with a different set of electrical measurements.

In this variation, a plurality of the occlusion indicators may be generated by averaging or summing each set of electrical measurements to produce a respective plurality of occlusion indicators. In this approach, the number of occlusion indicators is the same as the number of sets of electrical measurements. This approach produces a plurality of averaged electrical measurements. This variation can provide information about an occlusion at different positions about the interventional device, namely in the vicinity of different device electrodes.

In a continuation of either variation of this first example, each averaged electrical measurement may be compared to a threshold value to determine or predict whether or not occlusion has occurred. Breaching the threshold may indicate that occlusion has occurred (elsewise, occlusion has not occurred and a leakage has occurred). The threshold value may be predetermined, or may be set based on an initial or calibration electrical measurement for the relevant device electrode.

As an example, an initial electrical measurement for the device electrode may be obtained by averaging a set of initial electrical measurements for the device electrode obtained before occlusion is attempted. The initial electrical measurement, or a scaled version thereof, may then be used as the threshold value. The initial electrical measurement may be scaled to produce the threshold value by multiplying said initial electrical measurement by a predetermined value, such as 1.1 or 1.2.

A set of initial electrical measurements for a device electrode contains electrical measurements between the device electrode and a plurality of external electrodes that are obtained before occlusion is attempted. The plurality of external electrodes associated with a set of initial electrical measurements is the same plurality of external electrodes associated with the set of electrical measurements for that device electrode.

In a second example, the electrical measurements may be processed to identify the presence or absence of a leak and (if present) the location of any leak, e.g., with respect to the interventional device. Identification of the presence or absence of a leak can be performed, for instance, by comparing the (averaged) electrical measurement(s) to a threshold value.

For the second example, the electrical measurements preferably one or more sets of electrical measurements, in which the device electrode in the electrode pair for each electrical measurement in the same set of electrical measurements is the same; and the external electrode in the electrode pair for each electrical measurement in the same set of electrical measurements is different.

In particular, the location of each external electrode may be predetermined or otherwise known, e.g., as the external electrodes can be positioned in predetermined locations (as is well established for obtaining an ECG or performing device tracking). Thus, the spatial relationship between the external electrodes may be known. By monitoring the electrical measurement between each of these external electrode and a same device electrode, a position of the device electrode can be determined using a trilateration technique. If a leak is detected at the device electrode, then the location of the leak can similarly be determined.

As another example, the location of the device electrode may be tracked using a different technique, e.g., an electrode tracking technique such as that described by WO 2022/144198 A1. If a leakage is detected in the vicinity of a particular device electrode (by processing of a particular set of one or more electrical measurements), then the determined location of the device electrode may be associated with the detected leakage in order to produce a location of the leakage. This determined location can be used as an occlusion indicator.

If the interventional device is simultaneously being tracked, e.g., using a device tracking technique, then the location of any leak with respect to the interventional device can be determined.

In this way, the occlusion indicator can indicate, if occlusion is absent resulting in a leakage, the location of the leakage in the anatomical cavity. The location of the leakage may be identified as the location of the device electrode associated with the occlusion indicator.

It will be appreciated that it is similarly possible (using a similar approach) to identify a location of an occlusion within the anatomical cavity.

Of course, a combination of any of the above described example approaches can be used. For instance, a determination as to whether occlusion occurs in vicinity of a particular device electrode can be determined by combining (e.g., summing or averaging) a set of electrical measurements for that device electrode (e.g., and processing the result). If occlusion failure is detected, indicating that there is a leak, then the same set of electrical measurements could be processed to determine a location or orientation of the leak with respect to the interventional device.

In some examples, each electrical measurement may undergo pre-processing before undergoing step 420. For instance, a pre-processing step may comprise a step 430 of normalizing or calibrating each electrical measurement. This approach allows the electrical measurements to be directly comparable to one other.

Step 430 may, for instance, comprising a step 431 of receiving an initial electrical measurement for each electrode pair, the initial electrical measurement being originally sampled before attempted occlusion occurs. An electrical measurement may then be obtained for each electrode pair, the electrical measurement being originally sampled after attempted occlusion occurs (e.g., the balloon is inflated and/or position). Thus, the initial electrical measurement may have been sampled at an earlier point in time than the electrical measurement. Put another way, each initial electrical measurement may be responsive to an electrical property (between the corresponding electrode pair) before attempted occlusion and each electrical measurement may be responsive to the same electrical property (between the corresponding electrode pair) after attempted occlusion.

Each electrical measurement may then be normalized with respect to the initial electrical measurement obtained for the same electrode pair. For instance, each electrical measurement may be divided by the initial electrical measurement.

This approach adjusts the electrical measurements to be on a same scale, to improve the relevance and compatibility between the electrical measurements.

The method 400 may further comprise outputting the generated occlusion indicator(s) in a step 440. Step 400 may, for instance, comprise controlling a display or user interface to provide a visual representation of the occlusion indicator(s). Step 440 may comprise storing the occlusion indicator(s) in a memory or storage unit. In some examples, step 440 comprises providing the occlusion indicator to a further processing system for further processing.

Embodiments make use of a processing system. The processing system can be implemented in numerous ways, with software and/or hardware, to perform the various functions required. A processor is one example of a suitable processing module for a processing system which employs one or more microprocessors that may be programmed using software (e.g., microcode) to perform the required functions. A processing module for a processing system may however be implemented with or without employing a processor, and also may be implemented as a combination of dedicated hardware to perform some functions and a processor (e.g., one or more programmed microprocessors and associated circuitry) to perform other functions.

Examples of processing system components (e.g., a processing module) that may be employed in various embodiments of the present disclosure include, but are not limited to, conventional microprocessors, application specific integrated circuits (ASICs), and field-programmable gate arrays (FPGAs) .

In various implementations, a processor, processing module or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

**Claims**

1. An occlusion detection system for detecting occlusion of an anatomical cavity of a subject by an interventional device, which carries one or more device electrodes, using two or more external electrodes positioned on the surface of the subject, the occlusion detection system comprising:

   an input interface configured to receive at least two electrical measurements, each electrical measurement being responsive to a change in an electrical property between a different electrode pair compared to any other electrical measurement, where each electrode pair comprises a device electrode and an external electrode; and
   a data processor configured to process the at least two electrical measurements to generate an occlusion indicator that indicates the presence or absence of occlusion of the anatomical cavity by the interventional device.

2. The occlusion detection system of claim 1, wherein the electrical property is an impedance between the electrode pair.

3. The occlusion detection system of claim 1, wherein the electrical property is a voltage difference between the electrode pair.

4. The occlusion detection system of claim 1, wherein the electrical property is an impedance difference, being a difference between:

   an impedance between the device electrode of the electrode pair and a reference electrode; and
   an impedance between the external electrode of the electrode pair and the reference electrode.

5. The occlusion detection system of any of claims 1 to 4, wherein:

   the at least two electrical measurements comprises one or more sets of electrical measurements;
   the device electrode in the electrode pair for each electrical measurement in the same set of electrical measurements is the same; and
   the external electrode in the electrode pair for each electrical measurement in the same set of electrical measurements is different.

6. The occlusion detection system of claim 5, wherein the one or more sets of electrical measurements comprises two or more sets of electrical measurements, and wherein the device electrode in the electrode pairs of different sets of electrical measurements is different.

7. The occlusion detection system of any of claims 5 or 6, wherein the occlusion indicator indicates, if occlusion is absent resulting in a leakage, the location of the leakage in the anatomical cavity.

8. The occlusion detection system of claim 5, wherein:

   the location of each external electrode on the surface of the subject relative to one another is predetermined; and
   the data processor is configured to determine, if occlusion is absent resulting in a leakage, a location of the leakage in the anatomical cavity, with respect to the two or more external electrodes, using a trilateration technique.

9. The occlusion detection system of any of claims 1 to 8, wherein the at least two electrical measurements comprises at least four electrical measurements.

10. The occlusion detection system of any of claims 1 to 9, wherein the occlusion indicator indicates the amplitude of any leakage past the interventional device in the anatomical cavity.

11. The occlusion detection system of any of claims 1 to 10, wherein the data processor is configured to generate the occlusion indicator by performing a process that includes:

    monitoring the electrical measurements over a period of time; and
    generating the occlusion indicator responsive to changes in the electrical measurements over the period of time.

12. The occlusion detection system of any of claims 1 to 11, wherein the data processor is configured to generate the occlusion indicator by performing a process that includes summing or averaging the electrical measurements.

13. The occlusion detection system of any of claims 1 to 12, wherein:

the input interface is configured to receive, for each electrical measurement, an initial electrical measurement sampled at an earlier point in time; and

the data processor is configured to normalize each electrical measurement using the corresponding initial electrical measurement.

14. A computer-implemented method for detecting occlusion of an anatomical cavity of a subject by an interventional device, which carries one or more device electrodes, using two or more external electrodes positioned on the surface of the subject, the computer-implemented method comprising:

receiving at least two electrical measurements, each electrical measurement being responsive to a change in an electrical property between a different electrode pair compared to any other electrical measurement, where each electrode pair comprises a device electrode and an external electrode; and

processing the at least two electrical measurements to generate an occlusion indicator that indicates the presence or absence of occlusion of the anatomical cavity by the interventional device.

15. A computer program product comprising computer program code means which, when executed on a computing device having a processing system, cause the processing system to perform all of the steps of the computer-implemented method of claim 14.

FIG. 1

FIG. 2

300

Interventional
Device    100

210    External
Electrodes

310

Signal
Processor

351

350

353    352

Memory    320

360

FIG. 3

400

410 Receive electrical
measurements

430

431 Receive initial electrical
measurements

432 Normalize electrical
measurements

420 Generate occlusion
indicator(s)

440 Output occlusion
indicator(s)

FIG. 4

Europäisches
Patentamt
European
Patent Office
Office européen
des brevets

# EUROPEAN SEARCH REPORT

Application Number

EP 22 19 8917

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/001338 A1 (KONINKLIJKE PHILIPS NV [NL]) 7 January 2021 (2021-01-07) | 1-15 | INV.<br>A61B5/00 |
| Y | * page 25, line 9 - page 32, line 29 *<br>* figure 7 *<br>----- | 10 | ADD.<br>A61B18/00 |
| X | EP 3 922 169 A1 (KONINKLIJKE PHILIPS NV [NL]) 15 December 2021 (2021-12-15) | 1-9, 11-15 | |
| Y | * paragraph [0097] - paragraph [0098] *<br>* paragraph [0201] *<br>----- | 10 | |

TECHNICAL FIELDS
SEARCHED        (IPC)

A61B

The present search report has been drawn up for all claims

| Place of search | Date of completion of the search | Examiner |
|---|---|---|
| The Hague | 3 March 2023 | Van Dop, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 8917

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2023

| Patent document cited in search report | | Publication date | Patent family member(s) | | Publication date |
|---|---|---|---|---|---|
| WO 2021001338 | A1 | 07-01-2021 | CA | 3142455 A1 | 07-01-2021 |
| | | | CN | 114340531 A | 12-04-2022 |
| | | | EP | 3993695 A1 | 11-05-2022 |
| | | | JP | 2022539220 A | 07-09-2022 |
| | | | US | 2022354566 A1 | 10-11-2022 |
| | | | WO | 2021001338 A1 | 07-01-2021 |
| EP 3922169 | A1 | 15-12-2021 | NONE | | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6002955 A

- WO 2022144198 A1